# EUROPÄISCHE PATENTSCHRIFT

(11) **EP 2 254 475 B1**
(45) Veröffentlichungstag und Bekanntmachung des Hinweises auf die Patenterteilung: **10.08.2011**
(21) Anmeldenummer: 09711189.2
(22) Anmeldetag: 12.02.2009
(51) Int. Cl.: A61B 6/14, G06T 7/00, G06T 15/00, G06T 5/00

(54) **VERFAHREN ZUR ERSTELLUNG EINER SCHICHTAUFNAHME**
METHOD FOR GENERATING A TOMOGRAM
PROCEDE DE CREATION D'UNE TOMOGRAPHIE

(30) Priorität: 12.02.2008 DE 102008008733; 15.04.2008 US 71143 P
(43) Veröffentlichungstag der Anmeldung: 01.12.2010
(73) Patentinhaber: Sirona Dental Systems GmbH, 64625 Bensheim (DE)
(72) Erfinder: ULRICI, Johannes, 64293 Darmstadt (DE); HELL, Erich, 89537 Giengen (DE); BECKHAUS, Christian, 64297 Darmstadt (DE)
(74) Vertreter: Sommer, Peter
(86) Internationale Anmeldenummer: PCT/EP2009/051608
(87) Internationale Veröffentlichungsnummer: WO 2009/101123

(56) Entgegenhaltungen:
- WO-A-2006/127416
- DE-A1- 4 133 066
- DE-A1-102005 055 896
- JP-A- 2001 061 834
- JP-A- 2003 175 031
- US-A1- 2003 235 265
- US-A1- 2004 066 877
- US-A1- 2005 237 324
- US-B1- 6 493 415
- TOHNAK S ET AL: "Synthesizing panoramic radiographs by unwrapping dental CT data" ENGINEERING IN MEDICINE AND BIOLOGY SOCIETY, 2006. EMBS '06. 28TH ANNUAL INTERNATIONAL CONFERENCE OF THE IEEE, IEEE, PISCATAWAY, NJ, USA, 30. August 2006 (2006-08-30), Seiten 3329-3332, XP031390350 ISBN: 978-1-4244-0032-4

## Beschreibung

### Technisches Gebiet

Die Erfindung betrifft ein Verfahren zur Erstellung einer Schichtaufnahme, insbesondere einer PanoramaSchichtaufnahme (PAN-Aufnahmen), ausgehend von einem Röntgenstrahlenabsorptionswerte aufweisenden, digitalen 3D-Volumen, das beispielsweise durch Rekonstruktion aus Röntgen-Projektionsaufnahmen erzeugt sein kann. Derartige Schichtaufnahmen werden für die zahnärztliche Diagnose herangezogen.

Unter Tomographie versteht man ganz allgemein die Abbildung einer zweidimensionalen Schicht eines dreidimensionalen Objektes. Eine besondere Art, eine zweidimensionale Schicht zu erzeugen, ohne 3D-Daten zu verwenden, ist die Verwischungstomographie. Die Verwischungstomographie zeichnet sich dadurch aus, dass Punkte in einer sogenannten scharfen Schicht des Objektes bei einer Verschiebung eines Films und eines Strahlers bezüglich des Objektes immer auf derselben Stelle des Films und somit scharf abgebildet werden. Punkte außerhalb der scharfen Schicht werden hingegen auf dem Film als Verwischungskurven abgebildet, die den mechanischen Bewegungen von Strahler und Film bezüglich des Objektes entsprechen.

Der Unterschied zu einem 3D-tomographischen Verfahren besteht darin, dass dort bei der Berechnung einer Schicht eine Projektion von 3D auf 2D erzeugt wird, so dass alle Objektebenen senkrecht zur Projektionsachse gleichberechtigt dargestellt werden, ohne dass eine Ebene als scharfe Schicht besonders ausgezeichnet ist, etwa durch eine Überlagerung.

### Stand der Technik

Aus der EP 0 279 294 A1 ist eine zahnärztliche Röntgendiagnostikeinrichtung zur Erstellung von Panorama-Schichtaufnahmen vom Kiefer eines Patienten bekannt. Das beschriebene Verfahren erlaubt die Darstellung einer Schicht im Objekt, deren Mittelpunkt normalerweise der Kiefer des Patienten ist. Für die Erstellung der Panoramaschichtaufnahme wird dabei eine Strahler-Detektor-Anordnung um ein aufzunehmendes Objekt herum bewegt und die CCD-Detektorzeilen werden mit einer gegenüber der Bewegungsgeschwindigkeit geänderten Frequenz ausgetaktet, um eine Schichtaufnahme mit Verwischung der außerhalb der scharfen Schicht liegenden Anteile zu erstellen.

Zwar ist es möglich, anstatt einer Bilderzeugung in einer vorgegebenen scharfen Schicht durch entsprechendes Austakten von Bildinformation aus dem Detektor Einzelaufnahmen zu erstellen und diese entsprechend einer gewünschten Schichtlage erst nachträglich zu einer Panoramaschichtaufnahme zu überlagern. Aufgrund der vergleichsweise geringen Breite des normalerweise verwendeten Detektors werden jedoch stets nur Teilbereiche des aufzunehmenden Objekts erfasst und es ist nicht möglich, aus diesen Teilbereichen ein 3D-Volumen des aufzunehmenden Objekts zu erstellen. Bei der Verwendung von diesen sogenannten full frame Sensoren wird eine hohe Anzahl von Einzelaufnahmen erstellt. Die Anzahl der Einzelaufnahmen entspricht der während des normalen PAN-Umlaufs erfolgten Zeilentakte, was zu mehreren tausend Einzelaufnahmen führt.

Mit im Markt erhältlichen dentalen Röntgengeräten nach der Art eines digitalen Cone Beam Volumen-Tomographen (DVT) werden ein Strahler und ein Detektor in einer vorgegebenen x-y-Ebene gemeinsam um den Kopf bewegt. Die Bewegung erfolgt so, dass sich der Strahler und der Detektor um eine im aufzunehmenden Objekt liegende, in z-Richtung verlaufende Drehachse dreht. Aus verschiedenen Strahler- und Detektorpositionen auf dieser Umlaufbahn werden während des Umlaufs, der mindestens einen Winkel von 180° überdeckt, Projektionsaufnahmen gemacht, aus welchen anschließend ein 3D-Volumen berechnet wird. Dieses 3D-Volumen enthält Informationen über ortsabhängige Röntgenstrahlenabsorptionswerte bzw. daraus abgeleitete Bilddaten.

Aus diesen Werten wird eine Panoramaaufnahme erstellt, die jedoch keine herkömmliche Panoramaschichtaufnahme ist. Dazu wird der Verlauf und die Dicke der anzuzeigenden Schicht innerhalb des 3D-Volumens eingestellt oder ausgewählt und die in paralleler Durchleuchtung senkrecht zur Schicht summierten Röntgenstrahlenabsorptionswerte werden nach einer Aufbereitung als Bilddaten angezeigt. Dies ist beispeilsweise aus der Dissertation "Vergleich der Strahlenexposition bei der Digitalen-Volumen-Tomographie der Panoramaschichtaufnahme und der Computertomographie" vorgelegt von Christoph Schnelle, Medizinische Fakultät der Heinrich-Heine-Universität Düsseldorf, 2001, Seiten 1 bis 5 und dem Artikel "Das 3D-Röntgensystem GALILEOS" von Ch. Scheifele und P.A. Reichart, ZWR - Das deutsche Zahnärzteblatt 2007, Seiten 615 bis 617 bekannt.

Hier entsteht zwar ein Röntgenbild mit einem Bildeindruck, welcher zu einer mit einem Orthopantomographen erzeugten Panoramaschichtaufnahme ähnlich ist, allerdings werden Strukturen außerhalb der scharfen Schicht nicht berücksichtigt und es findet keine Verwischung statt.

Aus der DE 41 33 066 A1 ist ein Verfahren zur Erstellung einer Schichtaufnahme in Form eines Panoramabilds aus einem Röntgenstrahlenabsorptionswerte aufweisenden digitalen 3D-Volumen bekannt. Die erstellte Panoramaschichtaufnahme enthält Informationen entlang einer vorgegebenen gekrümmten tomographischen Bahn, wobei sie verschwommen sein kann.

Auch die US 2003/0235265 A1, die US 2005/0237324 A1, die US 2004/0066877 A1, die JP 2001-61834 A, die JP 2003-175031 A, die DE 10 2005 055 896 A1 und der Artikel von S. Tohnak et al.: "Synthesizing panoramic radiographs by unwrapping dental CT data", Proceedings of the 28th IEEE EMBS Annual International Conference, New York City, USA, 30. August - 3. September 2006, S. 3329-3332, ISBN 978-1-4244-0032-4 beschreiben das Erzeugen einer Schichtaufnahme aus einem digitalen 3D-Volumen.

Aus der WO 2006/127416 A2 ist ein Verfahren zur Berechnung und Optimierung einer Panoramaschichtaufnahme aus einzelnen Röntgenaufnahmen eines Objekts bekannt.

Die durch die beschriebenen Verfahren bereitgestellten, digital erzeugten Schichtaufnahmen ähneln herkömmlichen Panoramaschichtaufnahmen. Eine scharfe Schicht innerhalb von Verwischungsanteilen, wie sie sich bei einer klassischen Panoramaschichtaufnahme ausbildet, weist allerdings keine der beschriebenen Schichtaufnahmen auf.

Aufgrund der langjährigen Diagnosepraxis der Benutzer und der trotz Verwischung in üblichen PAN-Aufnahmen nach wie vor noch enthaltenen Bildinformationen kann es jedoch wünschenswert sein, auch in dem Fall, in dem ein durch Röntgen erhaltenes 3D-Volumen vorhanden ist, ein Röntgenbild bereitzustellen, das dem einer herkömmlichen Panoramaschichtaufnahme entspricht.

### Darstellung der Erfindung

Der Grundgedanke der Erfindung zur Bereitstellung einer Schichtaufnahme auch bei Röntgengeräten, die ein 3D-Volumen erzeugen, liegt in der Berechnung der Schichtaufnahme aus dem zuvor erzeugten 3D-Volumen, indem das verwischungstomographische Verfahren eines Ortopantomographen am 3D-Volumen nachgebildet wird und nicht indem eine Schicht aus dem 3D-Volumen extrahiert wird.

Bei dem erfindungsgemäßen Verfahren zur Erstellung einer Schichtaufnahme aus einem Röntgenstrahlenabsorptionswerte aufweisenden, digitalen 3D-Volumen, das insbesondere zur Erstellung einer dentalen Röntgen-Panoramaschichtaufnahme geeignet ist, wird das 3D-Volumen mit einer virtuellen Röntgenquelle virtuell durchstrahlt und das virtuell entstehende Bild mit einem virtuellen Detektor aufgenommen. Die virtuelle Röntgenquelle und der virtuelle Detektor werden unter Ausbildung einer scharfen Schicht mit einem Verwischungsanteil an dem aufzunehmenden Objekt virtuell vorbeibewegt.

Der virtuelle Aufnahmeablauf zur Aufnahme des 3D-Volumens als Objekt und das Zusammenwirken der virtuellen Röntgenquelle und des virtuellen Detektors können dabei einem herkömmlichen Ablauf zur Erstellung einer digitalen Schichtaufnahme mit einem Verwischungsanteil entsprechen.

Vorteilhafterweise können Strukturen, insbesondere anatomische Strukturen wie bspw. die Wirbelsäule, die bei einer Schichtaufnahme zu einer Schattenbildung führen und daher störend wirken, vor der Erstellung der simulierten Schichtaufnahme aus dem berechneten 3D-Volumen entfernt werden, etwa mittels eines Suchalgorithmus. Dadurch, dass die Schichtaufnahme erst nach Entfernung dieser Strukturen simuliert wird, kann eine Verbesserung der Bildqualität erreicht werden, da diejenigen Strukturen, die vom Betrachter noch berücksichtigt werden sollen, deutlicher herausgestellt werden.

Dies gilt sowohl für die unmittelbaren Informationen über die Zähne, aber auch für die Informationen, die gegenüber den bislang aus dem 3D-Volumen berechneten Panoramaaufnahmen wie bei einer herkömmlichen PAN-Aufnahme zusätzlich angezeigt werden.

Es kann vorgesehen sein, den Strahlenweg zu verändern, etwa an Stelle eines Strahlenfächers, der von einem Fokus in der Röntgenquelle ausgeht, eine Durchstrahlung mit einem Strahlenbündel aus parallelen Strahlen vorzunehmen, etwa um Verzerrungen der Größe in z-Richtung zu vermeiden.

Vorteilhafterweise kann der Verlauf der scharfen Schicht der berechneten Schichtaufnahme variiert werden. Für herkömmliche Schichtaufnahmen ist die Lage der scharfen Schicht für eine vorgegebene Position im Objekt nicht variabel.

Besonders vorteilhaft ist es, wenn der Abstand der scharfen Schicht zum virtuellen Fokus bzw. zum virtuellen Detektor senkrecht zu einer Fokus-Detektor-Umlaufebene veränderbar ist. Dadurch ist es möglich, die Aufnahme den anatomischen Strukturen wie bspw. schräg stehende Zähne anzupassen. Zwar wird hier die Standardaufnahme abgeändert, die übrigen Bildinformationen bleiben aber erhalten.

Vorteilhafterweise könnte über eine Veränderung der simulierten Detektorbreite in Verbindung mit einer Auffächerung des Strahlenfächers und der simulierten Umlaufgeschwindigkeit von Röntgenquelle und Detektor die Dicke der dargestellten scharfen Schicht variiert werden, um andere Informationen zur Anzeige zu bringen.

Besonders vorteilhaft kann die Schichtaufnahme eine Panorama-Schichtaufnahme sein und die virtuelle Röntgenquelle und der virtuelle Detektor werden anstelle um einen Patientenkopf virtuell um das 3D-Volumen bewegt. Der Abstand der virtuellen Röntgenquelle und des virtuellen Detektors zur scharfen Schicht, die Breite des virtuellen Detektors und die Verwischungsanteile können dabei einer Standard-Panoramaschichtaufnahme entsprechen. Auch der Strahlwinkel kann hier berücksichtigt sein.

Darüber hinaus können unterschiedliche Abstände zwischen der virtuellen Röntgenquelle, dem virtuellen Detektor und dem jeweiligen Drehmittelpunkt des Strahlenwegs verändert werden. Da sich die auf dem Markt befindlichen Geräte zur Erstellung von Panoramaschichtaufnahmen baulich unterscheiden, weisen auch die von den jeweiligen Geräten bereitgestellten Panoramaschichtaufnahmen jeweils gerätespezifische Besonderheiten auf. Es ist möglich, diese gerätespezifischen Besonderheiten zumindest teilweise bei der Erzeugung der Panoramaschichtaufnahme aus dem 3D-Volumen zu berücksichtigen.

Weiterhin kommt als Schichtaufnahme mit Verwischungsanteil auch eine Einzelzahnaufnahme in Betracht, etwa bei transversalen Schichtaufnahmen.

Wesentlich ist, dass alle Veränderungen der Schichtaufnahme ohne die Erstellung einer weiteren Röntgenaufnahme allein auf der Grundlage der vorhandenen Projektionsaufnahmen zur Erzeugung des 3D-Volumens vorgenommen werden können.

Gemäß einer Weiterbildung kann vor der Durchführung der virtuellen Schichtaufnahme darüber hinaus mittels eines Suchalgorithmus aus dem 3D-Volumen eine automatische Bestimmung der Lage einer scharfen Schicht im Objekt anhand charakteristischer anatomischer Strukturen geschaffen werden, etwa durch automatisches Ermitteln des Kieferkammbogens und dort vorliegender Zähne. Ist die Lage festgelegt, kann die virtuelle Aufnahme erfolgen. Anders als bei realen Aufnahmen kann sichergestellt werden, dass individuelle anatomische Besonderheiten stets mit berücksichtigt werden und nicht außerhalb einer vorgegebenen, standardisierten scharfen Schicht liegen.

Aus praktischer Sicht ist es derzeit sinnvoll, wenn das 3D-Volumen vor der Simulation der Schichtaufnahme bereits vollständig berechnet wurde. Es wäre jedoch auch möglich, dass die Simulation der Erstellung der Schichtaufnahme bereits ausgehend von den ursprünglichen Projektionsaufnahmen unabhängig von der Rekonstruktion des 3D-Volumens oder als Teil der Rekonstruktion erfolgt. Anhand der aus den Projektionsaufnahmen erhaltenen Informationen wird dann die Schichtaufnahme mit Verwischungsanteilen berechnet.

### Kurze Beschreibung der Zeichnungen

In der Zeichnung ist ein Ausführungsbeispiel der Erfindung dargestellt. Es zeigen:
- Fig. 1: ein Prinzipbild der virtuellen Aufnahmesituation eines 3D-Volumens zur Erstellung einer Panorama- schichtaufnahme mit einer scharfen Schicht,
- Fig. 2: eine nach dem Prinzip aus Fig. 1 erstellte Schichtaufnahme,
- Fig. 3: ein Prinzipbild für die Erstellung einer Panora- maaufnahme nach dem Stand der Technik,
- Fig. 4: eine nach dem Stand der Technik aus Fig. 3 er- stellte Aufnahme,
- Fig.: 5A,B eine in einer z-Richtung veränderte Lage der scharfen Schicht,
- Fig. 6: eine Aufnahmesituation mit einem schmalen und mit einem breiten Detektor.

### Ausführungsbeispiel der Erfindung

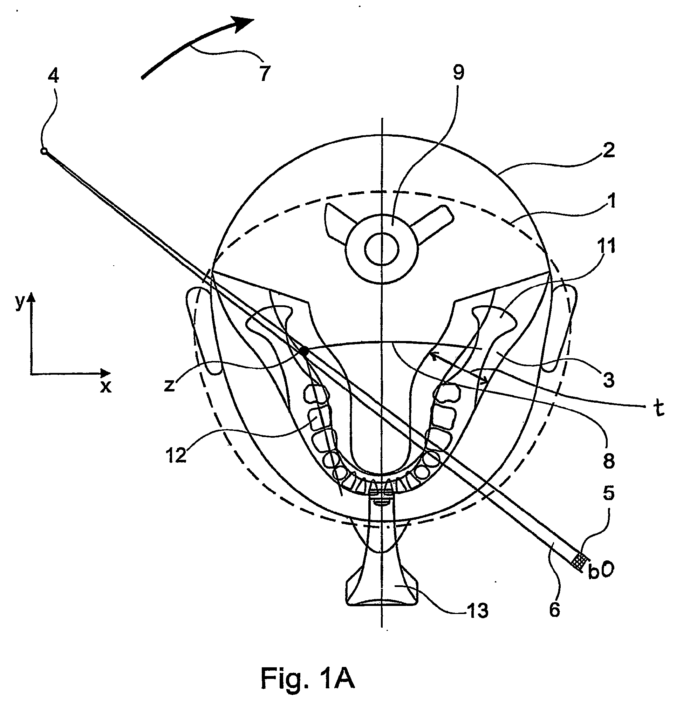

In Fig. 1A ist ein 3D-Volumen 1 eines Patientenkopfs 2 als zu untersuchendes Objekt dargestellt, das eine Abbildung eines Kieferbogens 11 und mehrerer Zähne 12 in Form von Informationen über Röntgenabsorptionswerte bzw. daraus gewonnenen Bilddaten enthält. Das 3D-Volumen 1 kann der gesamte Patientenkopf 2 oder wie dargestellt nur ein Teil davon sein, wobei das 3D-Volumen 1 nicht vollständig im Patientenkopf 2 liegen muss. Ausgehend von diesem 3D-Volumen 1 wird das erfindungsgemäße tomographische Verfahren mit Verwischung erläutert, wobei eine in dem 3D-Volumen 1 des Patientenkopf 2 verlaufende scharfe Schicht 3 nachgebildet wird. Die scharfe Schicht 3 entspricht einen Bereich mit einer gewissen Ausdehnung, einer Breite t, und kann zum Zweck der Vereinfachung auch durch eine Linie dargestellt werden.

Das 3D-Volumen 1 ist idealerweise so bestimmt, dass es alle für die Erstellung einer Panoramaschichtaufnahme erforderlichen anatomischen Merkmale enthält. Darüber hinaus können auch weitere anatomische Bereiche, etwa Weichteile wie Nase und Lippen im 3D-Volumen 1 enthalten sein.

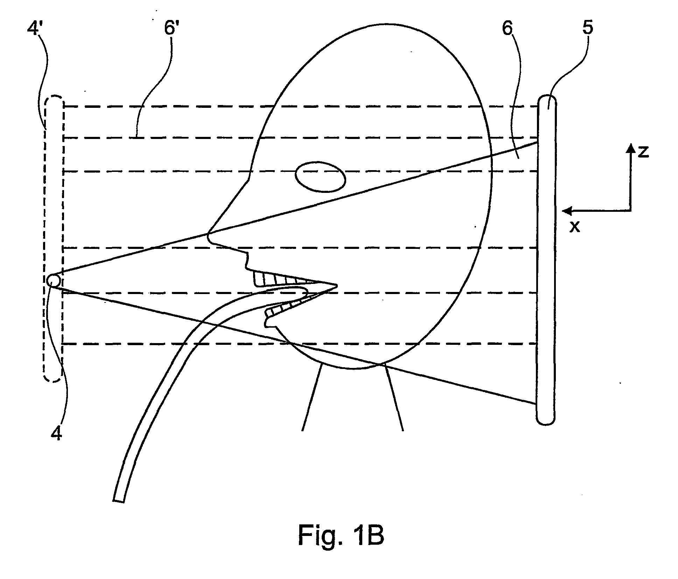

Das in Fig. 1 dargestellte 3D-Volumen 1 mit Röntgenstrahlenabsorptionswerten wird virtuell mit einer im vorliegenden Ausführungsbeispiel außerhalb des 3D-Volumens 1 angeordneten virtuellen Röntgenquelle 4 mit einem virtuellen Fokus durchstrahlt und mit einem ebenfalls außerhalb des 3D-Volumens 1 angeordneten virtuellen Detektor 5 mit einer Breite b0 werden virtuell abgeschwächte Strahlen eines virtuellen Strahlfächers 6 erfasst.

Für diese Aufnahme können beispielsweise die Röntgenstrahlenabsorptionswerte entlang des Strahlenwegs eines Strahls des virtuellen Strahlenfächers 6 mit einer bestimmten Querschnittsfläche von der virtuellen Röntgenquelle 4 zu dem virtuellen Detektor 5 addiert werden, was dann dem Signalwert eines Pixels eines wirklichen Detektors entspricht. Dabei wird nach Bedarf die nichtlineare Absorptionsfunktion für Röntgenstrahlen mit berücksichtigt.

Die virtuelle Röntgenquelle 4 und der virtuelle Detektor 5 können auch innerhalb des 3D-Volumens 1 angeordnet sein. Zwar kann damit eine Standard-PAN-Aufnahme nicht in üblicher Weise nachgebildet werden, die Bildeigenschaften dieser PAN-Simulation können jedoch anstelle des bislang verwendeten Panoramabilds verwendet werden.

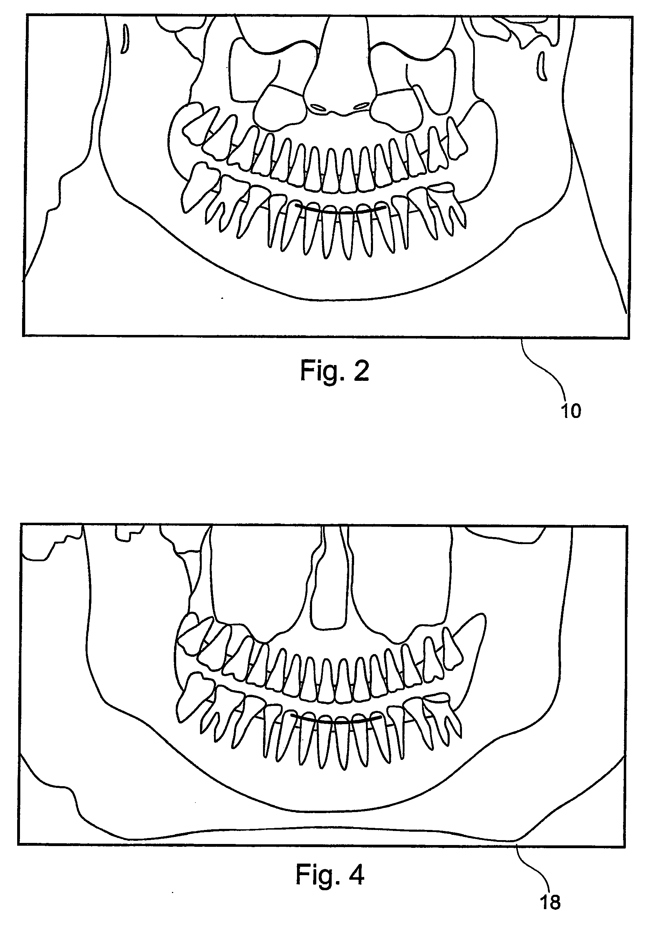

Mittels eines simulierten Umlaufs 7 der virtuellen Röntgenquelle 4 und des virtuellen Detektors 5 mit einer simulierten Umlaufgeschwindigkeit um ein Drehzentrum Z innerhalb des aufzunehmenden Objekts, dem 3D-Volumen 1, der dem für die Erstellung herkömmlicher PAN-Aufnahmen verwendeten Umlauf um den Kieferbogen 11 entspricht, wird eine simulierte Panoramaschichtaufnahme (Fig. 2) erzeugt. Dabei folgt das Drehzentrum Z während des simulierten Umlaufs 7 einer Kurve 8, wie es aus dem Stand der Technik für herkömmliche PAN-Aufnahmen bekannt ist. Zur Verdeutlichung ist ein virtueller Aufbiss 13 dargestellt, der den Patientenkopf 2 positioniert, wenn der Patient aufbeisst. Der Aufbiss 13 legt dabei auch eine Okklusalebene fest, die hier in der X-Y-Ebene liegt.

Während des simulierten Umlaufs 7 wird der virtuelle Detektor 5 dann entsprechend der Ansteuerung der echten Detektoren virtuell so ausgetaktet, d.h. die Bilddaten erzeugt, dass eine scharfe Schicht 3 mit einer Dicke t und eine Verwischung außerhalb der scharfen Schicht 3 erzeugt wird.

Beim Takten eines realen CCD-Detektors wird nämlich das gesamte auf dem Detektor aufgenommene Bild um eine Zeile verschoben. Erfolgt dieses Takten entgegen der Bewegungsrichtung des Röntgenquellen-Detektorsystems, werden Objektpunkte in der scharfen Schicht 3 im Bild immer auf den gleichen Punkt abgebildet, während Objektpunkte außerhalb der scharfen Schicht über das Bild wandern und so verschmieren.

Im 3D-Volumen 1 vorhanden unerwünschte Strukturen 9, etwa die Wirbelsäule oder der gegenüberliegende Kieferkammbogen, können ausgeblendet werden, bevor die simulierte PAN-Aufnahme erstellt wird.

In Fig. 1B ist ein Schnitt entlang des virtuellen Strahlenfächers 6 aus Fig. 1A schematisch dargestellt. Es ist zu erkennen, dass der virtuelle Strahlenfächer 6 auch in dieser X-Z-Ebene ausgehend vom Fokus der virtuelle Röntgenquelle 4 zum virtuelle Detektor 5 hin aufgeweitet ist.

Anstelle des virtuellen Strahlenfächers 6 kann auch eine virtuelle Durchstrahlung mit einem Strahlenbündel aus parallelen Strahlen 6' vorgenommen werden, wobei dann der Fokus der Röntgenquelle gedanklich entsprechend groß ausgebildet ist und parallele Strahlen abstrahlt. Anders als in der gegenständlichen Erzeugung einer Aufnahme ist diese rechnerische Erstellung einer Panoramaschichtaufnahme ohne weiteres möglich.

In Fig. 2 ist eine mit dem Verfahren erzeugte Schichtaufnahme 10 dargestellt und es ist für den Fachmann ohne weiteres erkennbar, dass es von seinem Informationsgehalt einer herkömmlichen Panoramaschichtaufnahme angenähert ist, wobei die Wirbelsäule entfernt wurde.

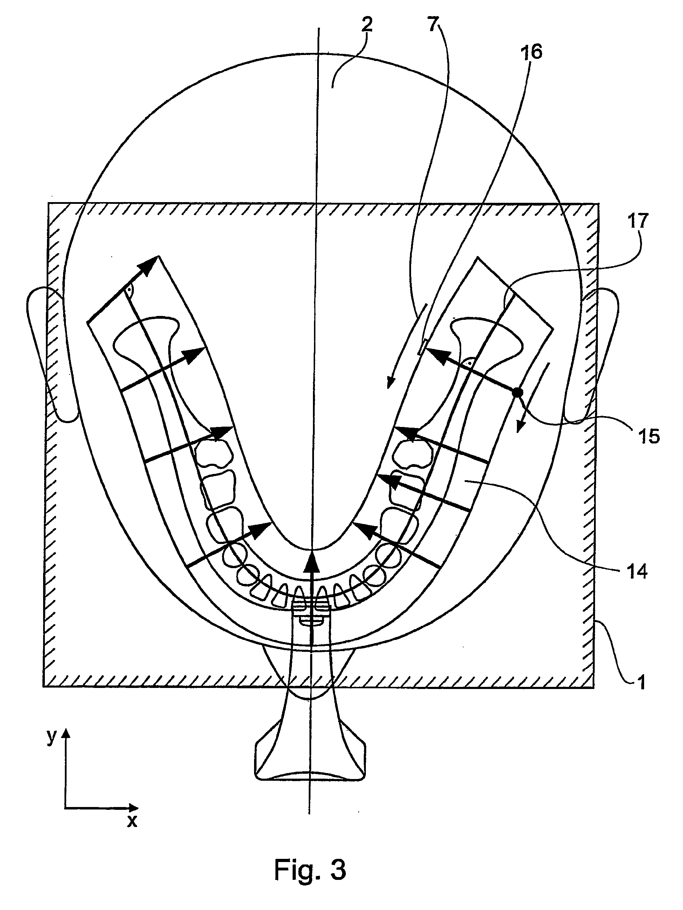

In Fig. 3 ist ein Prinzipbild für die Erstellung einer Panoramaaufnahme nach dem Stand der Technik dargestellt. Ausgehend von einem im digitalen 3D-Volumen 1 des Patientenkopfs 2 festgelegten Teilbereich 14, der von seiner räumlichen Ausdehnung her gesehen die scharfe Schicht 3 aus Fig. 1 beinhaltet, erfolgt eine virtuelle Durchstrahlung nur dieses Teilbereichs 14, das restliche 3D-Volumen 1 wird nicht berücksichtigt. Eine virtuelle Röntgenquelle 15 und ein virtueller Detektor 16 werden senkrecht zum Verlauf des Teilbereichs 14 an diesem entlang einer Bahn 17 bewegt und die entstehenden Projektionen werden in einer Gesamtaufnahme 18 als Panoramaaufnahme dargestellt, siehe Fig. 4.

Die in Fig. 4 dargestellte Aufnahme 18 ist jedoch für den Fachmann keine Schichtaufnahme im eigentlichen Sinn, da die Verwischungsanteile der Bildinformationen außerhalb der scharfen Schicht fehlen.

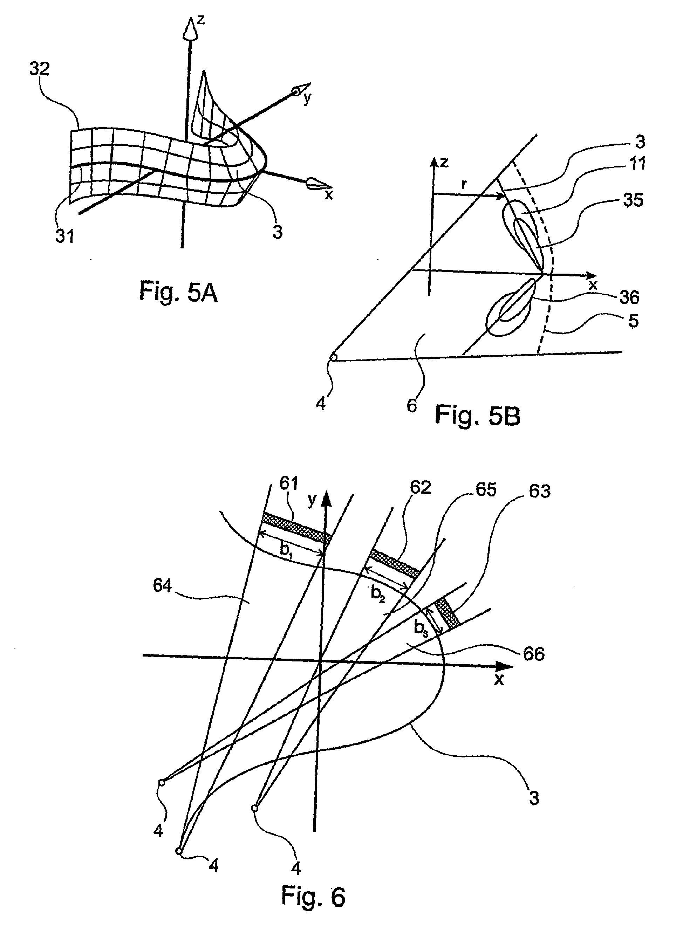

In Fig. 5A ist anhand einer Mittelebene der Scharfen Schicht 3 dargestellt, auf welche Art und Weise die Lage der scharfen Schicht in z-Richtung veränderbar ist. Die Mittelebene der scharfen Schicht 3 verläuft dem Kieferkammbogen folgend in X-Y-Richtung gekrümmt und variiert im dargestellten Beispiel darüber hinaus auch in Z-Richtung. Dieser Verlauf ist dann geeignet, wenn Zähne 35, 36 des Patienten gegenüber der X-Y-Ebene geneigt sind, im Detail dargestellt in Fig. 5B.

Dabei fällt die X-Y-Ebene mit der Okklusalebene des Patientenkopfes 2 zusammen und die Linie 31 entspricht der Mittelebene der scharfen Schicht in der Okklusalebene. Die Linie 32 entspricht der Mittelebene der scharfen Schicht oberhalb der Okklusalebene, also in Z-Richtung nach oben versetzt.

In Fig. 5B ist der Verlauf der scharfen Schicht 3 im Bereich von in einem Kieferbogen 11 befindlichen Zähnen 35, 36 als Schnitt in der X-Z-Ebene dargestellt. Es ist zu erkennen, dass die Mittelebene der scharfen Schicht in Z-Richtung dem Verlauf der Zähne 35, 36 folgt, dass die scharfe Schicht 3 also gekrümmt ist und sich damit ihr Abstand r zur Z-Achse in der Z-Richtung ändert.

Während der virtuellen Durchleuchtung ist die virtuelle Röntgenquelle 4 auf der Innenseite der Zähne 35, 36 angeordnet und der virtuelle Strahlenfächer 6 durchstrahlt den Kieferbogen 11 von innen nach außen. Der virtuelle Detektor 5 ist selbst ebenfalls gekrümmt ausgebildet, er kann aber auch gerade und/oder in einem größeren Abstand zur Mittelebene der scharfen Schicht 3 angeordnet sein.

In Fig. 6 ist eine Prinzipskizze dargestellt, bei der die Detektorbreite veränderbar und damit eine Beeinflussung der Dicke t der scharfen Schicht möglich ist.

Dargestellt sind drei verschiedene virtuelle Detektoren 61, 62, 63 mit einer Breite b1 bis b3 und einem entsprechend auf die Detektorbreite eingestellten virtuellen Strahlenfächer 64 bis 66 einer virtuellen Röntgenquelle 4. Mit einer derartigen Anordnung kann die Dicke t der scharfen Schicht 3, die hier nur als Linie dargestellt ist, während des simulierten Umlaufs variiert werden. In herkömmlichen Röntgengeräten zur Erstellung von Panoramaschichtaufnahmen ist dies so nicht möglich. Bei herkömmlichen Panoramaschichtaufnahmen kann die Breite des Detektors je nach Hersteller verschieden sein, sodass durch eine anpassbare Detektorbreite herstellerspezifische Standardaufnahmen bereitgestellt werden können.

Weiterhin können auch herstellerspezifische Strahlwinkel für den von der Röntgenquelle ausgehenden Strahlenfächer berücksichtigt werden, um herstellerbedingte Besonderheiten nachzubilden.

Neben Panorama-Schichtaufnahmen lassen sich auch transversale Schichtaufnahmen (TSA-Aufnahme) erstellen, wobei hier die Bewegung relativ zum Objekt zwar anders gestaltet ist, gleichwohl aber eine Verwischung stattfindet, die eine scharfe Schicht erzeugt.

Die Lage der scharfen Schicht bei der PAN-Aufnahme oder bei der TSA-Aufnahme kann frei wählbar oder für vorgegebenen Positionen vorwählbar sein, etwa um eine erste Anpassung an Größenverhältnisse bereitzustellen. Weiterhin kann auch eine automatische Bestimmung der Lage anhand anatomischer Strukturen erfolgen.

### Bezugszeichenliste

- 1: digitales 3D-Volumen
- 2: Patientenkopf
- 3: scharfe Schicht
- 4: virtuelle Röntgenquelle
- 5: virtueller Detektor
- 6: virtueller Strahlenfächer
- 7: simulierter Umlauf der virutellen Röntgenquelle 4 und des virtuellen Detektors 5
- 8: Kurve, die der Drehzentrum Z während eines simulierten Umlaufs 7 folgt
- 9: unerwünschte Strukturen
- 10: Schichtaufnahme
- 11: Kieferbogen
- 12: Zähne
- 13: Aufbiss
- 14: Teilbereich des digitalen 3D-Volumens 1
- 15: virtuelle Röntgenquelle
- 16: virtueller Detektor
- 17: Bahn entlang derer die virtuelle Röntgenquelle 15 und der virtuelle Detektor 16 bewegt werden
- 31: Mittelebene der scharfen Schicht 3 in der Okklusalebe ne
- 32: Mittelebene der scharfen Schicht 3 oberhalb der Okklu salebene
- 35: Zahn
- 36: Zahn
- 61: virtueller Detektor
- 62: virtueller Detektor
- 63: virtueller Detektor
- 64: virtueller Strahlenfächer entsprechend der Breite b1 des virtuellen Detektors 61
- 65: virtueller Strahlenfächer entsprechend der Breite b2 des virtuellen Detektors 62
- 66: virtueller Strahlenfächer entsprechend der Breite b3 des virtuellen Detektors 63
- b1: Breite des virtuellen Detektors 5 bzw. 61
- b2: Breite des virtuellen Detektors 62
- b3: Breite des virtuellen Detektors 63
- r: Abstand der scharfen Schicht zur Z-Achse
- t: Dicke der scharfen Schicht 3
- Z: Drehzentrum des simulierten Umlaufs 7

## Patentansprüche

1. Verfahren zur Erstellung einer Schichtaufnahme (10) nach Art einer dentalen Röntgen-Panoramaschichtaufnahme aus einem Röntgenstrahlenabsorptionswerte aufweisenden, digitalen 3D-Volumen (1), wobei das 3D-Volumen (1) als aufzunehmendes Objekt mit einer virtuellen Röntgenquelle (4; 15) virtuell durchstrahlt und das virtuell entstehende Bild mit einem virtuellen Detektor (5; 16; 61, 62, 63) aufgenommen wird, **dadurch gekennzeichnet, dass** die virtuelle Röntgenquelle (4; 15) und der virtuelle Detektor (5; 16; 61, 62, 63) unter Ausbildung einer scharfen Schicht (3) mit einem Verwischungsanteil an dem aufzunehmenden Objekt virtuell vorbeibewegt werden.

2. Verfahren nach Anspruch 1, **dadurch gekennzeichnet, dass** vor der Erstellung der Schichtaufnahme (10) aus dem 3D-Volumen (1) für die Bildgebung unerwünschte Strukturen (9) entfernt werden.

3. Verfahren nach Anspruch 1 oder 2, **dadurch gekennzeichnet, dass** vor oder während der Erstellung der Schichtaufnahme (10) die Lage der scharfen Schicht (3) anpassbar ist.

4. Verfahren nach Anspruch 3, **dadurch gekennzeichnet, dass** der Abstand der scharfen Schicht (3) zur virtuellen Röntgenquelle (4; 15) bzw. zum virtuellen Detektor (5; 16; 61, 62, 63) senkrecht zu einer Röntgenquelle-Detektor-Umlaufebene veränderbar ist.

5. Verfahren nach einem der Ansprüche 1 bis 4, **dadurch gekennzeichnet, dass** die Breite (b0; b1, b2, b3) des virtuellen Detektors (5; 16; 61, 62, 63), die Auffächerung des virtuellen Strahlenfächers (6; 64, 65, 66) und die simulierte Umlaufgeschwindigkeit der virtuellen Röntgenquelle (4; 15) und des virtuellen Detektors (5; 16; 61, 62, 63) veränderbar ist, um die Dicke (t) der aufgenommenen scharfen Schicht (3) zu beeinflussen.

6. Verfahren nach einem der Ansprüche 1 bis 5, **dadurch gekennzeichnet, dass** die Schichtaufnahme (10) eine Panorama-Schichtaufnahme ist.

7. Verfahren nach Anspruch 6, **dadurch gekennzeichnet, dass** der Abstand der virtuelle Röntgenquelle (4; 15) und des Detektors (5; 16; 61, 62, 63) zur scharfen Schicht (3), die Breite (b0; b1, b2, b3) des Detektors (5; 16; 61, 62, 63) und die Verwischungsanteile einer Standard-Panoramaschichtaufnahme entsprechen.

8. Verfahren nach einem der Ansprüche 1 bis 5 **dadurch gekennzeichnet, dass** die Schichtaufnahme (10) eine Einzelzahnaufnahme ist.

9. Verfahren nach einem der Ansprüche 1 bis 8, **dadurch gekennzeichnet, dass** vor der Durchführung der Schichtaufnahme (10) mittels eines Suchalgorithmus aus dem 3D-Volumen (1) anhand charakteristischer anatomischer Strukturen eine automatische Bestimmung der Lage der scharfen Schicht (3) im Objekt festgelegt wird.

## Claims

1. A method for creating a tomographic image (10) in the manner of a dental X-ray panoramic tomographic image from a digital 3D volume (1) exhibiting X-ray absorption values, wherein said 3D volume (1) forming the object to be scanned is transradiated virtually using a virtual X-ray source (4; 15) and the virtually produced image is recorded by a virtual detector (5; 16; 61, 62, 63), **characterized in that** said virtual source of X-rays (4; 15) and said virtual detector (5; 16; 61, 62, 63) are moved virtually past the object to be scanned with the formation of a sharp layer (3) having a blurred area.

2. The method as defined in claim 1, **characterized in that** structures (9) undesirable for imaging are removed from said 3D volume (1) prior to the creation of said tomographic image (10).

3. The method as defined in claim 1 or claim 2, **characterized in that** the position of said sharp layer (3) can be adjusted before or during the creation of said tomographic image (10).

4. The method as defined in claim 3, **characterized in that** the distance of said sharp layer from said virtual source of X-rays (4; 15) or from said virtual detector (5; 16; 61, 62, 63) can be modified in a direction normal to an X-ray source/detector plane of rotation.

5. The method as defined in any one of claims 1 to 4, **characterized in that** the width (b0; b1, b2, b3) of said virtual detector (5; 16; 61, 62, 63), the spread of the radiation fan beam (6; 65, 65, 66), and the simulated speed of rotation of said virtual source of X-rays (4; 15) and of said virtual detector (5; 16; 61, 62, 63) can be modified in order to control the thickness of the sharp layer (3) created.

6. The method as defined in any one of claims 1 to 5, **characterized in that** said tomographic image (10) is a panoramic tomographic image.

7. The method as defined in claim 6, **characterized in that** the distance of said source of X-rays (4; 15) and of said detector (5; 16; 61, 62, 63) from said sharp layer (3), the width (b0; b1, b2, b3) of said detector (5; 16; 61, 62, 63), and the blurred areas correspond to a standard panoramic tomographic image.

8. The method as defined in any one of claims 1 to 5, **characterized in that** said tomographic image (10) is an image of a single tooth.

9. The method as defined in any one of claims 1 to 8, **characterized in that** prior to the creation of said tomographic image (10), the position of said sharp layer (3) in the object is automatically determined from said 3D volume (1) by means of a search algorithm for finding characteristic anatomical structures.

## Revendications

1. Procédé de réalisation d'une tomographie (10) à la manière d'une tomographie panoramique en radiographie dentaire à partir d'un volume 3D numérique (1) présentant des valeurs d'absorption de rayons X, dans lequel le volume 3D (1) en tant qu'objet à photographier est virtuellement radiographié par une source de rayons X virtuelle (4 ; 15), et l'image produite virtuellement est acquise par un détecteur virtuel (5 ; 16 ; 61, 62, 63), **caractérisé en ce que** la source de rayons X virtuelle (4 ; 15) et le détecteur virtuel (5 ; 16 ; 61, 62, 63) sont déplacés virtuellement devant l'objet à photographier en réalisant une couche nette (3) avec une partie estompée.

2. Procédé selon la revendication 1, **caractérisé en ce que** des structures (9) indésirables pour la formation d'image sont éliminées avant de réaliser la tomographie (10) à partir du volume 3D (1).

3. Procédé selon la revendication 1 ou 2, **caractérisé en ce que** la position de la couche nette (3) peut être adaptée avant ou pendant la réalisation de la tomographie (10).

4. Procédé selon la revendication 3, **caractérisé en ce** la distance entre la couche nette (3) et la source de rayons X virtuelle (4 ; 15) ou le détecteur virtuel (5 ; 16 ; 61, 62, 63) est variable perpendiculairement à un plan de rotation de la source de rayons X/ du détecteur.

5. Procédé selon l'une quelconque des revendications 1 à 4, **caractérisé en ce que** la largeur (b0 ; b1, b2, b3) du détecteur virtuel (5 ; 16 ; 61, 62, 63), l'ouverture en éventail de l'éventail de rayons virtuel (6 ; 64, 65, 66) et la vitesse de rotation simulée de la source de rayons X virtuelle (4 ; 15) et du détecteur virtuel (5 ; 16 ; 62, 62, 63) sont variables afin de jouer sur l'épaisseur (t) de la couche nette (3) photographiée.

6. Procédé selon l'une quelconque des revendications 1 à 5, **caractérisé en ce que** la tomographie (10) est une planigraphie panoramique.

7. Procédé selon la revendication 6, **caractérisé en ce que** la distance de la source de rayons X virtuelle (4 ; 15) et du détecteur (5 ; 16 ; 61, 62, 63) par rapport à la couche nette (3) correspond à la largeur (b0 ; b1, b2, b3) du détecteur (5 ; 16 ; 61, 62, 63) et que les parties estompées correspondent à une tomographie panoramique standard.

8. Procédé selon l'une quelconque des revendications 1 à 5, **caractérisé en ce que** la tomographie (10) est une prise de vue d'une seule dent.

9. Procédé selon l'une quelconque des revendications 1 à 8, **caractérisé en ce qu'**avant la réalisation de la tomographie (10) un algorithme de recherche permet de fixer à partir du volume 3D (1), à l'aide de structures anatomiques caractéristiques, une détermination automatique de la position de la couche nette (3) dans l'objet.
